(19) Europäisches Patentamt
European Patent Office
Office européen des brevets

(11) Publication number: **0 551 689 A2**

# (12) EUROPEAN PATENT APPLICATION

(21) Application number: **92300243.0**

(22) Date of filing: **10.01.92**

(51) Int. Cl.5: **C07K 7/64**, C07K 7/56, A61K 39/21

(30) Priority: **19.12.91 US 807943**

(43) Date of publication of application:
**21.07.93 Bulletin 93/29**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Bednarek, Maria A.**
**270 McFarlane Road No. 167**
**Colonia, NJ 07067(US)**
Inventor: **Tolman, Richard L.**
**29 Upper Warren Way**
**Warren, NJ 07059(US)**

(74) Representative: **Thompson, John Dr. et al**
**Merck & Co., Inc. European Patent**
**Department Terlings Park Eastwick Road**
**Harlow, Essex CM20 2OR (GB)**

## (54) Cyclic HIV principal neutralizing determinant (PNP) peptides.

(57) Cyclic Human Immunodeficiency Virus (HIV) Principal Neutralizing Determinant (PND) peptides, or peptides immunologically equivalent therewith, wherein the ring system of the cyclic peptide is formed by ring closure through a stable amide bond, are useful as analytical tools, as reagents in ELISA assays, or as reagents for making covalent conjugate immunogens. Conjugates containing these stable, cyclic peptides are useful for raising mammalian anti-peptide, anti-HIV, or HIV-neutralizing immune responses, and a composition containing such a conjugate may be used as a vaccine to prevent HIV-Disease, including Acquired Immune Deficiency Syndrome, (AIDS), or AIDS related complex, (ARC), or as an immunogen for treating humans afflicted with HIV-Diseases, such as AIDS or ARC.

EP 0 551 689 A2

## BACKGROUND OF THE INVENTION

Human Immunodeficiency virus (HIV) Principal Neutralizing Determinant (PND) peptides have been described which are capable of raising an anti-HIV immune response in mammals. The hypervariable region of the AIDS virus envelope glycoprotein, gp120, between amino acids 296 and 341 [according to the numbering scheme of Ratner et al., Nature 313, 277 (1985)] contains the amino acid tetramer -GlyProGlyArg- (-GPGR-) in most of the HIV isolates identified to date. In addition, a cysteine residue is generally found within about 20 amino acids on either side of this tetramer. In at least one common isolate, HIV IIIB, it is known that these cysteines are disulfide bonded. Thus, the intermediate amino acids, herein referred to as loop amino acids, are forced into a cyclic structure, with the -GPGR- being exposed at the loop-tip [Javaherian et al., PNAS USA 86, 6768, (1989)].

Peptide based efforts aimed at the induction of HIV neutralizing immune responses in mammals have generally been limited to the use of linear or disulfide bonded cyclic peptides. The linear peptides have the limitation that the recipient immune system is exposed to an epitope which constantly changes its three dimensional conformation in solution, while reliance on disulfide bonding to limit the number of conformations assumable by the linear PNDs is not completely satisfactory as disulfides are labile. Breakage of the disulfides results in the linear peptide having the attendant problems described above.

In addition to the problems associated with linear or disulfide bonded peptides noted above, an additional problem is that a great many different isolates of the HIV have been identified wherein sequence divergence of the PND has been noted. This diversity implies that if PNDs producing isolate specific immune responses are to be effective anti-HIV immunogens, then a mixture of PND peptides from different isolates may be the only solution to attainment of a broadly therapeutic or protective immunogen. In order to better characterize effective PND secondary structures, stable cyclics which avoid the use of labile disulfide bonding are needed. The cyclic peptides and the process for making these products disclosed in the instant invention satisfy this need.

Linear synthetic peptides have been prepared by a number of strategies conducted either in solution or on solid supports. Excellent texts covering the principles and techniques involved are: Principles of Peptide Synthesis, Bodanszky. M., Springer-Verlag (1984); Solid Phase Peptide Synthesis, Stewart J. M., Young, J. D., Pierce Chemical Company (2nd. ed. 1984); The Peptides, Gross, E., Meienhofer, J., Academic Press, Inc., (1979). In general, where cyclic peptides are required, disulfide bonded cyclics have been prepared by oxidation of linear synthetic peptides containing at least two sulfhydryls, for example two cysteines. The instant invention overcomes the problems associated with the use of labile disulfides by providing peptides having stable, amide-bonded cyclic structures.

Thus, this invention discloses novel HIV PND peptides having stable cyclic structures, a process for making, and a method of using such compounds. The stable cyclic HIV PND peptides, cPNDs, are prepared by amide bond formation between a free amino group on the amino terminal side of the loop amino acids and a free carboxyl group on the carboxy-terminal side of the loop amino acids, preferably the free carboxy terminus of the peptide. The cPNDs so formed are useful reagents for preparing covalent conjugate immunogens containing said cPNDs. Such conjugates are useful for raising mammalian anti-peptide, anti-HIV, or HIV-neutralizing immune responses. Compositions containing such conjugates may be used as a vaccine to prevent HIV-Disease, including Acquired Immune Deficiency Syndrome, (AIDS), or AIDS related complex (ARC) or as an immunogen for treating humans afflicted with AIDS or ARC. In addition, such conjugates provide a useful laboratory tool for analyzing the structure-function relationship involved in peptide elicitation of mammalian HIV-neutralizing immune responses.

## SUMMARY OF THE INVENTION

This invention is concerned with novel, stable, cyclic HIV PND peptides, having the structure:

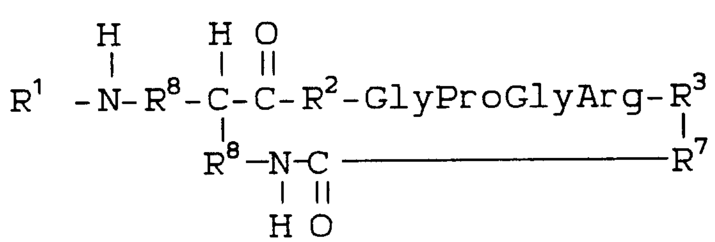

wherein

$R^1$ is:

a) hydrogen, or

b) an amino acid optionally including a marker amino acid selected from norleucine, ornithine, $\beta$-alanine, and gamma amino butyric acid;

$R^2$ is a bond, an amino acid, or a peptide of 2 to 17 amino acids;

$R^3$ is an amino acid or a peptide of 2 to 17 amino acids;

$R^7$ is:

a) a bond, or

b) lower heteroalkylene of formula $-(CH_2)_n-NH-$, wherein n is 1-20;

$R^8$ is a bond or $-(CH_2)_4-$; and

provided that $R^7$ must be $-(CH_2)_n-NH-$ if $R^2$ does not contain the group $-NH-(CH_2)_n-CO-$.

Peptides having this structure are prepared by cyclizing linear HIV PND peptides, which are made by known solid-phase chemical synthetic methods incorporating appropriate side-chain protection. Following synthesis, the linear HIV PND peptide is cleaved from the resin, and cyclized in solution by treatment with a reagent capable of mediating amide bond formation, preferably diphenylphosphorylazide (DPPA), benzotriazol-1-yloxytris(dimethylamino)phosphonium hexafluorophosphate (BOP), or similar reagent. A free amino group on one side of the loop amino acids and a free carboxyl group on the other side of the loop amino acids participate in formation of the cyclic structure. The amino group may be provided by the free amino terminus of the peptide, preferably by a norleucine which allows for easy peptide quantitation, by the free amino group of an amino-terminal isoglutamine (iQ), or by the $\epsilon$-amino or $\alpha$-amino group of a lysine on the amino-terminal side of the loop amino acids. The carboxyl may be provided by either the free peptide carboxy terminus or by the side chain of an acidic amino acid such as glutamate or aspartate.

The stable, amide-bonded cyclic peptides are useful as analytical tools, as reagents in ELISA assays, or as reagents for conjugation to an immunogenic carrier which may be comprised of polysaccharide, protein, both polysaccharide and protein, or any other material which confers enhanced immunogenicity on the cyclic peptides. Such conjugates are useful for inducing mammalian anti-peptide, anti-HIV, or HIV-neutralizing immune responses and for formulating vaccines to prevent HIV-Disease, including AIDS or ARC, or for treating humans afflicted with HIV-Diseases, such as AIDS of ARC.

## OBJECTS OF THE INVENTION

Accordingly, it is an object of this invention to provide novel, stable, and cyclic HIV PND peptides which are useful as reagents for preparing conjugates which may be used to elicit mammlian anti-peptide, anti-HIV, or HIV-neutralizing immune responses, or to prepare compositions for use as anti-HIV vaccines or as immunogens for treatment of humans afflicted with HIV-Diseases, including AIDS and ARC. Another object is to provide a process for the stable cyclization of HIV PND peptides through amide bonded structures.

## DEFINITIONS AND ABBREVIATIONS

| | |
|---|---|
| AA assay | amino acid analysis method wherein peptides or proteins are acid hydrolyzed to the free amino acids and then quantitated |
| Abu | $\gamma$-aminobutyric acid; |
| Acp | 6-aminocaproic acid; |
| Acm | acetamidomethyl; thiol protecting group |
| Aund | 11-aminoundecanoic acid; |
| activation | reaction of peptides, proteins, or polysaccharide moieties with a reagent capable of derivatizing the moiety in order to enable subsequent desirable reactions to occur |
| AIDS | Acquired Immune Deficiency Syndrome |
| amino acid | a molecule having both an acid and amino functional group; there are 20 common $\alpha$-amino acids with the general structure $H_2N-CHR-COOH$, wherein the R group defines the identity of the amino acid; also included in this definition are amino acids having the general structure $H_2N-(CH_2)_n-COOH$, wherein the R group is hydrogen, and n is 1-20; the amino acids may have either a D or L stereochemical form and unless specified otherwise, by the lower case one letter abbreviation, or by the prefix "D-" before an amino acid name, the amino acid is of the natural or L configuration; the names of the 20 common amino acids and the structure of the R group are identified herein in single-letter code according to the following table: |

| AMINO ACID NAME | 3-letter code | 1-letter code | side-chain (R) |
|---|---|---|---|
| Alanine | Ala | A | $-CH_3$ |
| Arginine | Arg | R | $-(CH_2)_3NHCHNH_2NH_2^+$ |
| Asparagine | Asn | N | $-CH_2CONH_2$ |
| Aspartic Acid | Asp | D | $-CH_2COOH$ |
| Cysteine | Cys | C | $-CH_2SH$ |
| Glutamic Acid | Glu | E | $-(CH_2)_2COOH$ |
| Glutamine | Gln | Q | $-(CH_2)_2CONH_2$ |
| Glycine | Gly | G | -H |
| Histidine | His | H | $-CH_2$-imidazole |
| Isoleucine | Ile | I | $-CH(CH_3)CH_2CH_3$ |
| Leucine | Leu | L | $-CH_2CH(CH_3)_2$ |
| Lysine | Lys | K | $-(CH_2)_4NH_3^+$ |
| Methionine | Met | M | $-(CH_2)_2SCH_3$ |
| Phenylalanine | Phe | F | $-CH_2$-Phenyl |
| Proline | Pro | P | -$\alpha$,N-trimethylene |
| Serine | Ser | S | $-CH_2OH$ |
| Threonine | Thr | T | $-CH(OH)CH_3$ |
| Tryptophan | Trp | W | $-CH_2$-indole |
| Tyrosine | Tyr | Y | $-CH_2$-phenyl-OH |
| Valine | Val | V | $-CH(CH_3)_2$ |

| | |
|---|---|
| antibody | a protein produced by mammalian B cells that is capable of binding a particular antigen |
| ARC | AIDS-Related Complex |
| AZT | Azidothymidine, an anti-AIDS compound |
| bigeneric spacer | a molecular chain or conjugate resulting from the reaction of separately derivatized partners; analytical degradation of the conjugate formed through this spacer allows release and quantitation of the spacer, providing a measure of the degree of covalent attachment |
| BOP | benzotriazol-1-yloxytris(dimethylamino)phosphonium hexafluorophosphate |
| capping | the elimination of reactive sites on a conjugate by reaction with small molecules |
| Cbz | benzyloxycarbonyl |
| conjugate | a complex of discrete chemical entities covalently bound one to the other, wherein at least one entity is a desired antigen (e.g. an HIV PND) and another entity is a carrier protein or carrier replacement |
| Core amino acids | those amino acids of an HIV PND which are essential for inducing HIV-neutralizing immune responses in a mammal |
| DPPA | diphenylphosphorylazide |
| ELISA | enzyme-linked immunosorbant assay |
| Fmoc | 9-fluorenylmethyloxycarbonyl |
| HIV | Human Immunodeficiency Virus, a member of the lentivirus group and the purported etiologic agent implicated in AIDS and |

| | |
|---|---|
| | related complexes; HIV is alternatively known as HTLV (Human T-cell Lymphocyto-trophic Virus), LAV (Lymphadenopathy Associated Virus), and ARV (AIDS Related Virus) |
| immunogen | a molecule useful as a stimulator of a mammalian immune response |
| immunologically equivalent peptides | cyclic or linear peptides having in common the function of eliciting HIV neutralizing immune responses in mammals, such as antibodies which are able to recognize the equivalent peptide epitopes |
| marker amino acid | an amino acid having a signal in the AA assay which is free of interference by signals generated by other peptide or protein amino acids, for example, norleucine, ornithine, β-alanine, gamma amino butyric acid |
| Mtr | 4-methoxy-2,3,6-trimethylphenyl sulfonyl |
| NEM | N-ethylmaleimide |
| OMPC | Outer Membrane Protein Complex of Neisseria meningitidis; used as an immunoenhancer and peptide carrier |
| peptide | a polymer of amino acids linked by amide (peptide) bonds |
| PEP | peptide |
| PND | Principal Neutralizing Determinant; the name attributed to peptidyl sequences capable of binding to HIV neutralizing antibodies and capable of raising HIV-neutralizing antibodies in a mammalian recipient upon inoculation with an immunogen containing the PND; for example, residues 296-341, or subfragments thereof, of HIV gp120 |
| PnPs6B | Streptococcus pneumoniae 6B capsular polysaccharide |
| PRO | an immunogenic protein |
| protein | a large peptide |
| PRP | Polyribosyl-ribitol phosphate |
| PSA | anionic polysaccharide, usually having repeat phosphate units in the monomer unit of the polymer |
| resins | solid support matrices for solid phase peptide synthesis<br>WANG:<br>4-(hydroxymethyl)phenoxymethyl linkage to copolystyrene-1%divinylbenzene resin, which is used for batch Fmoc solid phase peptide synthesis, with final 95% TFA cleavage from the resin and concomitant deprotection of acid sensitive side chain protecting groups;<br>SASRIN:<br>4-(hydroxymethyl)-3-methoxyphenoxymethyl linkage to copolystyrene-1%divinylbenzene resin, which is used for batch Fmoc solid phase peptide synthesis, with final 1% TFA/$CH_2Cl_2$ cleavage from the resin, leaving intact acid labile side chain protecting groups;<br>PEPSYN KA:<br>4-(hydroxymethyl)phenoxymethyl linkage to polyamide resin adsorbed on to KIESELGUHR, which is used for continuous flow column Fmoc solid phase peptide synthesis. Peptides are cleaved from the resin as described above for WANG resin;<br>PEPSYN KH:<br>4-(hydroxymethyl)-3-methoxymethyl linkage to polyamide resin adsorbed on to KIESELGUHR, which is used for Fmoc solid phase peptide synthesis. Side chain protected peptides are cleaved from the resin as described above for the SASRIN resin |
| "scaffold" | immunogen having multiple peptide epitopes built upon a spacer framework and which is attached to a carrier molecule |
| SCMHC | S-carboxymethyl homocysteamine, an acid-stable bigeneric spacer released by degradation of covalent conjugate im- |

munogens and quantifiable by AA assay

SCMC S-carboxymethyl cysteamine, an acid-stable bigeneric spacer released by degradation of covalent conjugate immunogens and quantifiable by AA assay

Z benzyloxycarbonyl

DETAILED DESCRIPTION OF THE INVENTION

This invention is concerned with novel, cyclic HIV PND peptides having the structure cPND:

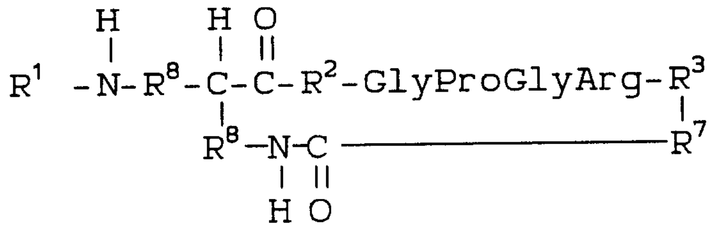

wherein

$R^1$ is:

a) hydrogen, or

b) an amino acid optionally including a marker amino acid selected from norleucine, ornithine, $\beta$-alanine, and gamma amino butyric acid;

$R^2$ is :

a) a bond, an amino acid, or a peptide of 2 to 17 amino acids, or

b) a peptide of between 2 to 17 amino acids;

$R^3$ is an amino acid or a peptide of 2 to 17 amino acids;

$R^7$ is:

a) a bond, or

b) lower heteroalkylene of formula $-(CH_2)_n-NH-$, wherein n is 1-20;

$R^8$ is a bond or $-(CH_2)_4-$; and

provided that $R^7$ must be $-(CH_2)_n-NH-$ if at least one amino acid constituting $R^2$ is not of the type of general formula: $-NH-(CH_2)_n-CO-$.

The term "amino acid" includes compounds having the following general structure: $NH_2(CH_2)_nCOOH$, wherein n is 1-20. When incorporated into a peptide, this type of amino acid is a divalent radical having the general structure: $-NH(CH_2)_nCO-$.

Hereinafter, amino acids $-R^2$-GPGR-$R^3$-, which go toward formation of the loop of a cyclic peptide, will be referred to as loop amino acids. Guidance as to the sequence of $R^2$ and $R^3$ is provided by LaRosa, et al., [Science 249, 932-935 (1990)], wherein the sequence surrounding the Gly Pro Gly Arg tetramer in HIV gp120 of 245 HIV isolates was examined. Thus, $R^2$ may be selected from any of the sequences found at the amino-terminal side of the tetramer, up to the cysteine, and $R^3$ may be selected from any of the sequences found at the carboxy-terminal side of the tetramer, up to the cysteine.

The term "nonlabile bond" means a covalent linkage, other than a labile disulfide bond, such as amide and thioether bonds. By using an appropriate bridge structure, the conformation of the loop structure of the cycle may be optimized allowing the fine tuning of the PND epitope to be presented to the immune system. For example, use of a bridge structure containing 2 carbons generates a "tighter" loop than when a $C_5$-containing bridge is used.

A preferred cyclic HIV PND of this invention has the structure:

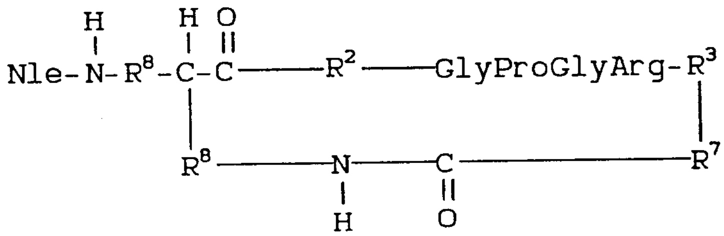

wherein:

$R^8$ is a bond or $-(CH_2)_4-$;

$R^7$ is a bond or $-(CH2)_n-NH-$, wherein n is 1-20;

$R^2$ consists of $X_nX_1X_2$, with $X_2$ closest to the GlyProGlyArg sequence;

$X_1$ is a constituent of $R^2$ selected from:

a) serine,
b) proline,
c) arginine,
d) histidine,
e) glutamine, and
f) threonine;

$X_2$ is a constituent of $R^2$ selected from:

a) isoleucine,
b) arginine,
c) valine,
d) methionine, and
e) $-NH-(CH_2)_nCO-$, wherein n is 1-20;

$X_n$ is a constituent of $R^2$ and is either a bond, an amino acid, or a peptide of 2 to 15 amino acids, with 0 or one of said amino acids having the structure:

$-NH-(CH_2)_n-CO-$, wherein n is 1-20;

Likewise, $R^3$ is comprised of $X_3X_4X_m$, with $X_3$ closest to the GlyProGlyArg sequence,

$X_3$ is a constituent of $R^3$ selected from:

a) alanine,
b) arginine,
c) valine, and
d) leucine;

$X_4$ is a constituent of $R^3$ and is selected from:

a) phenylalanine,
b) isoleucine,
c) valine, and
d) leucine;

$X_m$ is a constituent of $R^3$ and is a bond, an amino acid, or a peptide of up to 15 amino acids, with 0 or one of said amino acids having the structure:

$-NH-(CH_2)_n-CO$, wherein n is 1-20; and

provided that $R^7$ must be $-(CH_2)_n-NH-$ if $R^2$ does not contain the group $-NH-(CH_2)_n-CO-$.

The novel cyclic peptides of this invention are prepared in essentially two phases: First, the linear peptide is prepared, for example on an ABI-431A peptide synthesizer, by known solid phase peptide synthetic chemistry, for example using Fmoc chemistry and appropriately side-chain protected Fmoc-amino acids as reagents.

Second, the linear peptide is cleaved from the resin and cyclized in solution by allowing the free amino terminus of the peptide, the free amino group of the amino terminus to be amide bonded to a free carboxyl group on the carboxy-terminal side of the loop amino acids through DPPA, BOP, or similar reagent mediated peptide bond formation.

Products obtained may be characterized by fast atom bombardment-mass spectrometry [FAB-MS], reverse phase HPLC, amino acid analysis, or nuclear magnetic resonance spectroscopy (NMR).

The cyclic peptides of this invention may be used as reagents in ELISA assays, and provide a tool for analyzing peptide structure function relationships when HIV-neutralizing antibodies are used in the assay. Alternatively, the cyclic peptides of the invention may be conjugated to a carrier, for example a polysaccharide, an immunogenic protein, or a combination of any material capable of enhancing the immunogenicity of the peptide. For example, peptides of this invention were conjugated to a carrier comprising the outer membrane protein complex (OMPC) of Neisseria meningitidis b. Examples are provided below wherein such conjugates are prepared.

For use as a vaccine, the peptide of this invention should be administered in a conjugated state at an immunologically effective concentration. Preferably, between 1μg and 1mg, and most preferably about 100-300μg of the conjugated peptide is administered to a mammal intramuscularly, intravenously, or subcutaneously. The conjugate is preferably administered after being adsorbed onto a suitable adjuvant, for example, aluminum hydroxide gel (see Example 6-8).

Conjugate immunogens of these peptides are able to elicit anti-peptide immune responses in a mammal. For example, cPND955 conjugated to OMPC raised antibodies in rabbits at 12 weeks post-immunization. In 2 out of 4 of these rabbits, the antibodies raised were HIV neutralizing antibodies.

Likewise, cPND978 conjugated to OMPC raised anti-peptide antibodies in rabbits, and, in 3 out of 4 rabbits tested, the antibodies raised were HIV neutralizing antibodies.

In each case, the conjugate was prepared by maleimidating the cyclized peptide and reacting the maleimidated peptide with thiolated OMPC. The conjugate thus formed was absorbed onto alum and a dose of 100-300 μg of the conjugate was administered intramuscularly at 0, 4, and 8 weeks.

The following examples are provided to more particularly demonstrate how to make and use the cyclic peptides of this invention. However, the examples provided are not to be construed so as to limit the scope of the invention.

EXAMPLE 1

Preparation of cPND724, [Seq. Id. : 1 : ]

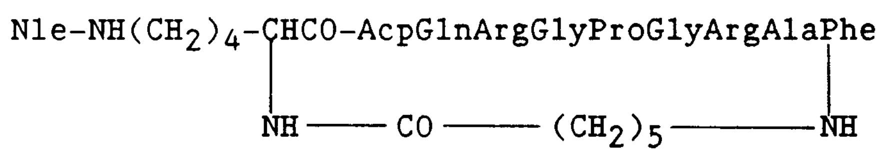

The linear peptide, [Seq. Id. : 5 : ]

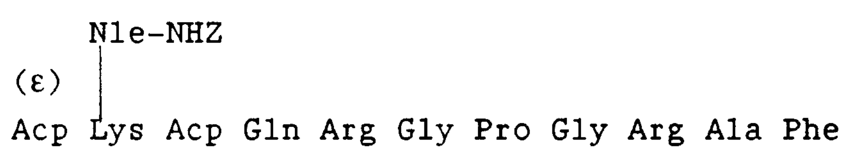

was synthesized using Fmoc-Lys(ε Nle-Z)-OH, Fmoc chemistry and single coupling on an ABI-431 solid phase synthesizer, at 0.25 mmolar scale. The peptide was cleaved from the resin with trifluoroacetic acid (TFA) and anisole, 20°C, 4.5 hours. About 0.4 g of crude material was purified on a Waters Delta Prep system, and the peak fraction was dried. Mass spectrometry analysis gave a molecular weight of 1473, which is consistent with the calculated molecular mass.

The linear peptide (0.34g, 0.22 mmol) was dissolved in 200 mL water, along with diisopropylethylamine (DIEA) (0.12 mL, 0.66 mmol), BOP (111 mg, 0.25 mmol), and HOBt (38 mg, 0.25 mmol). An additional aliquot of BOP (100 mg, 0.25 mmol) was added after analysis of an aliquot revealed the presence of residual linear peptide. After further incubation at room temperature, a 1 mL aliquot was evaporated, redissolved in 50% acetic acid, and analyzed by HPLC. All of the linear material was converted to cyclic peptide according to this analysis. Therefore, the entire reaction was dried, redissolved in 50% acetic acid, and purified by HPLC. The sample was loaded on a $C_{18}$ column equilibrated with aq. 0.1% TFA, and the peptide eluted over 60 minutes by gradient up to 80% $CH_3CN$/0.1% TFA.

Peak HPLC fractions were combined, dried, and hydrogenated over a palladium catalyst. The catalyst was filtered off and the sample was dried, and then dissolved in 50% acetic acid. The sample was repurified by HPLC, and peak fractions eluting over 70 minutes in an aq. 0.1% TFA - 70% $CH_3CN$/0.1% TFA gradient were pooled and dried. Mass spectroscopic analysis gave a molecular weight of 1336, which is consistent with the calculated mass for cPND724.

EXAMPLES 2-4

Preparation of cPND978, cPND955, and cPND979:

Using essentially the same procedure as Example 1, but varying the primary amino acid sequence, the following compounds were prepared, and the molecular weight confirmed by mass spectroscopic analysis:

EX # : Name : Mass : Structure

Ex #2 : cPND978 : 1223 : [Seq. Id. : 2 :]

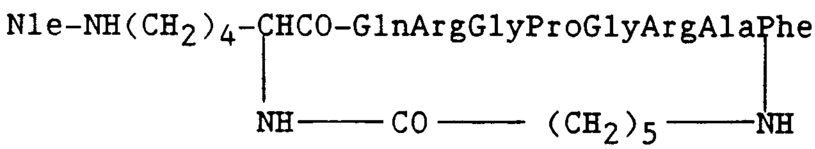

Ex #3 : cPND955 : 1302 : [Seq. Id. : 3 :]

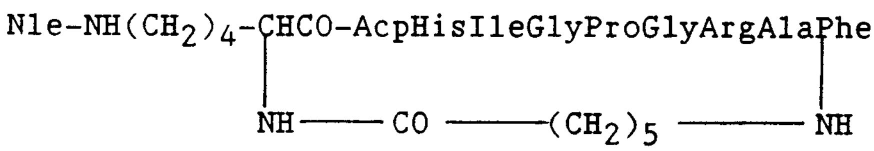

Ex #4 : cPND979 : 1475 : [Seq. Id. : 4 :]

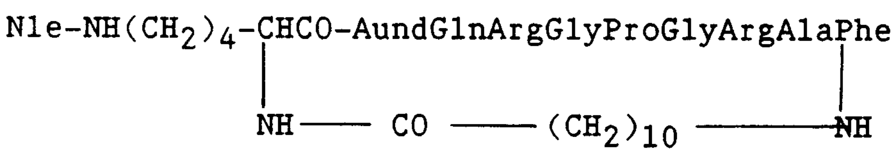

EXAMPLE 5

Preparation of OMPC-SH:

10 mililiters of OMPC (3.2 mg/ml) was centrifuged, at 43,000 ram, 4°C for 2 hours. The OMPC pellet was resuspended in 8 ml of thiolating solution (prepared by mixing 85 mg EDTA, 17 mg DTT, and 46 mg N-acetyl homocysteine thiolactone in 10 ml of pH 11 borate buffer). The thiolation reation was allowed to proceed at room temperature overnight, and the solution was then centrifuged at 43,000 rpm, 4°C for 2 hours. The OMPC-SH was resuspended in 10 ml of 0.01 M, pH 8 phosphate buffer, recentrifuged, and resuspended in 9.3 ml of 0.01 M, pH 8 phosphate buffer. An Ellman assay indicated a sulfhydryl titer of 0.496 $\mu$moles/ml.

EXAMPLE 6

Protocol for Inoculation of Animals with cPND-OMPC conjugates:

Alum was used as an adjuvant during the inoculation series. The inoculum was prepared by dissolving the coconjugate in physiologic saline at a final conjugate concentration of 300 µg/ml. Preformed alum (aluminum hydroxide gel) was added to the solution to a final level of 500 µg/ml aluminum. The conjugate was allowed to adsorb onto the alum gel for two hours at room temperature. Following adsorption, the gel with the conjugate was washed twice with physiologic saline and resuspended in saline to a protein concentration of 300 µg/ml.

Rabbits or African green monkeys were individually inoculated with three 300 µg doses or three 100 µg doses of the conjugate either adsorbed onto alum, or formulated with the Ribi adjuvant. Each dose was injected intramuscularly. The doses were delivered one month apart (week 0, 4 and 8). The animals were bled at intervals of two weeks. Serum samples were prepared from each bleed to assay for the development of specific antibodies as described in the subsequent examples.

EXAMPLE 7

Analysis of Sera for Anti-Peptide IgG Antibodies:

Each serum sample is analyzed by enzyme-linked immunoadsorbent assay (ELISA). Polystyrene microtiter plates were coated with 0.5 µg per well of the synthetic peptide (not conjugated to OMPC) in phosphate-buffered physiological saline (PBS) at 4°C. Each well was then washed with PBS containing 0.05% TWEEN-20 (PBS-T). Test serum, diluted serially in PBS-T, was added to the peptide-containing wells and allowed to react with the adsorbed peptide for one hour at 36°C. After washing with PBS-T, alkaline phosphatase-conjugated goat anti-human IgG was added to the test wells and was allowed to react for one hour at 36°C. The wells were then washed extensively in PBS-T. Each well received 0.1% p-nitrophenyl phosphate in 10% diethanolamine, pH 9.8, containing 0.5 mM $MgCl_2 \cdot 6H_2O$. The ensuing reaction was allowed to proceed at room temperature for 30 minutes, at which time it was terminated by the addition of 3.0 N NaOH.

The greater the interaction of antibodies in the test serum with the peptide substrate, the greater is the amount of alkaline phosphatase bound onto the well. The phosphatase enzyme mediates the breakdown of p-nitrophenyl phosphate into a molecular substance which absorbs light at a wavelength of 405 nm. Hence, there exists a direct relationship between the absorbance at 405 nm of light at the end of the ELISA reaction and the amount of peptide-bound antibody.

All the rabbits inoculated with the cPND-OMPC conjugates developed antibodies specifically capable of binding the peptide.

EXAMPLE 8

Analysis of Sera for Activity which Specifically Neutralizes HIV Infectivity:

Virus-neutralizing activity is determined with an assay described by Robertson et al., J. Virol. Methods 20: 195-202 (1988). The assay measures specific HIV-neutralizing activity in test serum. The assay is based on the observation that MT-4 cells, a human T-lymphoid cell line, are readily susceptible to infection with HIV and, after a period of virus replication, are killed as a result of the infection.

The test serum is treated at 56°C for 60 minutes prior to the assay. This treatment is required to eliminate non-specific inhibitors of HIV replication. Heat treated serum, serially diluted in RPMI-1640 cell culture medium, is mixed with a standard infection dose of HIV. The dose is determined prior to the assay as containing the smallest quantity of virus required to kill all the MT-4 cells in the assay culture after a period of 7-8 days. The serum-virus mixture is allowed to interact for one hour at 37°C. It then is added to $1.0 \times 10^5$ MT-4 cells suspended in RPMI-1640 growth medium supplemented with 10% fetal bovine serum. The cultures are incubated at 37°C in a 5% $CO_2$ atmosphere for 7 days.

At the end of the incubation period, a metabolic dye, DTT, is added to each culture. This dye is yellow in color upon visual inspection. In the presence of live cells, the dye is metabolically processed to a molecular species which yields a blue visual color. Neutralized HIV cannot replicate in the target MT-4 cells and therefore does not kill the cells. Hence, positive neutralization is assessed by the development of blue color following addition of the metabolic dye.

EXAMPLE 9

Preparation of Maleimido Propionly cPND955, MPP-cPND955:

cPND955, prepared according to Example 29, was maleimidated as follows:
To a cooled solution of cPND 955 triflouroactate salt, (9.5 mg, in 0.8 ml of 70% acetonitrile) was added 61 ul. of 0.5 M sodium bicarbonate solution followed by 2.3 mg. of maleimidopropionyl (N-Hydroxy) succinimide. The solution was stirred in ice for 1 hr. and the course of the reaction was followed by reverse phase HPLC (30% MeCN/0.1% TFA). The reaction was quenched with 2.3 ul of TFA and the peptide was isolated by preparative HPLC using gradient elution from 20 to 40% MeCN in 0.1% TFA over 30 minutes. The peak eluting between 21.6-30.6 minutes was collected, concentrated and lyophylized. Wt 9.3 mg.; FAB-MS, m/z 1454, M+H.

EXAMPLE 10

Conjugation of MPP-cPND955 with OMPC-SH:

To suspension of OMPC-SH (4.3 ml, 0 546 uM SH/ml) was added maleimidopropionic Acid (204 ul, 4.61 uM/ml; 0.4 eq.) with vigorous mixing. After ten minutes a solution of MPP'955 (268 ul, 5.23 uM/ml; 0.6 eq.) was added and the mixture was aged at room temperature for one hr. The suspension was dialysed vs 4 l. of pH 8, 0.01 M phosphate buffer overnight in the cold room and the dialysate transferred to a sterile tube. The final volume was 4.8 ml. Lowry protein = 3.68 mg/ml; Nle = 2.9 nm/ml; Peptide loading = 8.6%.

EXAMPLE 11

Evaluation of the Immunogenicity of Conjugated cPND955 in Rabbits:

OMPC-cPND955 prepared according to Example 51 was used to inoculate Rabbits intramuscularly at weeks 0, 4, and 8, (i.e. a similar protocol to that of Example 47 was used). Each dose contained 300 μg of the conjugate and emulsified in Freund's Adjuvant (complete adjuvant for the first dose; incomplete adjuvant for the subsequent doses).

ELISAs and virus-neutralization as says were performed as described in Examples 48 and 49. High titers of anti-peptide antibody was detected by ELISA assay, and high titers of HIV (MN isolate) neutralizing antibodies were detected in each of two rabbits inoculated with this conjugate.

EXAMPLE 12

Development of HIV neutralizing antibodies in response to immunization with Conjugated Peptides of this Invention:

Peptides of this invention were active in raising HIV neutralizing antibodies in rabbits or African green monkeys as described in Example 6 and analyzed as described in Examples 7, 8, and 11 as follows:

| Compound | Conjugated to: |
|---|---|
| cPND955 | OMPC |
| cPND978 | OMPC |

EXAMPLE 13

## Preparation of Neisseria meningitidis B11 Serotype 2 OMPC

### A. Fermentation

1. Neisseria meningitidis Group B11

A tube containing the lyophilized culture of Neisseria meningitidis (obtained from Dr. M. Artenstein, Walter Reed Army Institute of Research (WRAIR), Washington, D.C.) was opened and Eugonbroth (BBL) was added. The culture was streaked onto Mueller Hinton agar slants and incubated at 37°C with 5% $CO_2$ for 36 hours, at which time the growth was harvested into 10% skim milk medium (Difco), and aliquots were frozen at -70°C. The identity of the organism was confirmed by agglutination with specific antiserum supplied by WRAIR, and typing serum supplied by Difco.

A vial of the culture from the second passage was thawed and streaked onto 10 Columbia Sheep Blood agar plates (CBAB-BBL). The plates were incubated at 37°C with 5% $CO_2$ for 18 hours after which time the growth was harvested into 100 mL of 10% skim milk medium, aliquots were taken in 0.5 mL amounts and frozen at -70°C. The organism was positively identified by agglutination with specific antiserum, sugar fermentation and gram stain.

A vial of the culture from this passage was thawed, diluted with Mueller-Hinton Broth and streaked onto 40 Mueller-Hinton agar plates. The plates were incubated at 37°C with 6% $CO_2$ for 18 hours after which time the growth harvested into 17 mL of 10% skim milk medium, aliquotted in 0.3 mL amounts and frozen at -70°C. The organism was positively identified by Gram stain, agglutination with specific antiserum and oxidase test.

2. Fermentation and collection of cell paste

a. Inoculum Development- The inoculum was grown from one frozen vial of Neisseria memingitidis Group B, B-11 from above (passage 4). Ten Mueller-Hinton agar slants were inoculated, and six were harvested approximately 18 hours later, and used as an inoculum for 3 250 mL flasks of Gotschlich's yeast dialysate medium at pH 6.35. The $O.D._{660}$ was adjusted to 0.18 and incubated until the $OD_{660}$ was between 1 and 1.8. 1 mL of this culture was used to inoculate each of 5 2L. Erlenmeyer flasks (each containing 1 liter of medium; see below) and incubated at 37°C in a shaker at 200 rpm. The O.D. was monitored at hourly intervals following inoculation. 4 liters of broth culture, at an $O.D._{660}$ of 1.28 resulted.

70 Liter Seed Fermenter- Approximately 4 liters of seed culture was used to inoculate a sterile 70-liter fermenter containing about 40 liters of complete production medium (see below). The conditions for the 70-liter fermentation included 37°C, 185 rpm with 10 liters/minute air sparging and constant pH control at about pH 7.0 for about 2 hours. For this batch, the final $O.D._{660}$ was 0.732 after 2 hours.

800-Liter Production Fermenter

Approximately 40 liters of seed culture were used to inoculate a sterile 800 liter fermenter containing 568.2 liters of complete production medium (see below). The batch was incubated at 37°C, 100 rpm with 60 liters/minute air sparging and constant pH control at pH 7.0. For this batch, the final O.D. was 5.58 thirteen hours after inoculation.

3. Complete Medium for Erlenmeyer flasks and 70-and 800-liter fermenters

**Fraction A**

| | |
|---|---|
| L-glutamic acid | 1.5 g/liter |
| NaCl | 6.0 g/liter |
| $Na_2HPO_4$.anhydrous | 2.5 g/liter |
| $NH_4Cl$ | 1.25 g/liter |
| KCl | 0.09 g/liter |
| L-cysteine HCl | 0.02 g/liter |

**Fraction B (Gotschlich's Yeast Dialysate):**

1280 g of Difco Yeast Extract was dissolved in 6.4 liters of distilled water. The solution was dialyzed in 2 Amicon DC-30 hollow fiber dialysis units with three H10SM cartridges. 384 g $MgSO_4$.7-$H_2O$ and 3200 g dextrose were dissolved in the dialysate and the total volume brought to 15 liters with distilled water. The pH was adjusted to 7.4 with NaOH, sterilized by passage through a 0.22 $\mu$ filter, and transferred to the fermenter containing Fraction A.

For the Erlenmeyer flasks: 1 liter of Fraction A and 25 mL of Fraction B were added and the pH was adjusted to 7.0-7.2 with NaOH.

For the 70 liter fermenter: 41.8 liters of Fraction A and 900 mL of Fraction B were added and the pH was adjusted to 7.0-7.2 with NaOH.

For the 800 liter fermenter: 553 liters of Fraction A and 15.0 liters of Fraction B were added and the pH was adjusted to 7.1-7.2 with NaOH.

d. Harvest and Inactivation

After the fermentation was completed, phenol was added in a separate vessel, to which the cell broth was then transferred, yielding a final phenol concentration of about 0.5%. The material was held a room temperature with gentle stirring until the culture was no longer viable (about 24 hours).

e. Centrifugation

After about 24 hours at 4°C, the 614.4 liters of inactivated culture fluid was centrifuged through Sharples continuous flow centrifuges. The weight of the cell paste after phenol treatment was 3.875 kg.

**B. OMPC Isolation**

Step 1. Concentration and diafiltration

The phenol inactivated culture was concentrated to about 30 liters and diafiltered in sterile distilled water using 0.L micro-hollow fiber filters (ENKA).

Step 2. Extraction

An equal volume of 2X TED buffer [0.1 M TRIS 0.01 M EDTA Buffer, pH 8.5, with 0.5% sodium deoxycholate] was added to the concentrated diafiltered cells. The suspension was transferred to a temperature regulated tank for OMPC extraction at 56 °C with agitation for 30 minutes.

The extract was centrifuged at about 18,000 rpm in a Sharples continuous flow centrifuge at a flow rate of about 80 mL/minute, at about 4°C. The viscous supernatant was then collected and stored at 4°C. The extracted cell pellets were reextracted in TED buffer as described above. The supernatants were pooled and stored at 4°C.

Step 3. Concentration by Ultrafiltration

The pooled extract was transferred to a temperature regulated vessel attached to AG-Tech 0.1 micron polysulfone filters. The temperature of the extract was held at 25°C in the vessel throughout the concentration process. The sample was concentrated tenfold at an average transmembrane pressure of between 11 and 24 psi.

Step 4. Collection and Washing of the OMPC

The retentate from Step 3 was centrifuged at about 160,000 x g (35,000 rpm) at about 70°C in a continuous flow centrifuge at a flow rate between 300 to 500 mL/minute, and the supernatant was discarded.

The OMPC pellet was suspended in TED Buffer (190 mL buffer; 20 mL/g pellet) Step 2 and Step 4 were repeated twice (skipping Step 3).

Step 5. Recovery of OMPC Product

The washed pellets from Step 4 were suspended in 100 mL distilled water with a glass rod and a Dounce homogenizer to insure complete suspension. The aqueous OMPC suspension was then filter sterilized by passage through a 0.22 μ filter, and the TED buffer was replaced with water by diafiltration against sterile distilled water using a 0.1 μ hollow fiber filter.

EXAMPLE 14

Preparation of the N-(bromoacetyl)-6-aminocaproic derivative of OMPC, (BrAc-6-ACA-OMPC):

An OMPC solution (10 ml) (59 mg/ml) was centrifuged at 43K rpm, 4°c for 2 hours. The pellet was resuspended using a DOUNCE homogenizer in 6 ml of pH9 (KOLTHOFF) buffer and 1 milliliter of a N-(bromoacetyl)-6-aminocaproic acid p-nitrophenyl ester solution (85 mg/ml of acetonitrile) was added. The resultant mixture was agitated for 45 hours. Insoluble material was pelleted by a low speed centrifugation and the supernatant was then centrifuged at 43K rpm, 4°C for 2 hours. The pellet was resuspended on 10 ml of $H_2O$ and recentrifuged at 43K, 2 hours at 4°C. This pellet was resuspended in 10 ml of $H_2O$ affording the BrAc-6-ACA-OMPC. The amino acid analysis showed 268 nanomole/ml of -6-aminocaproic acid and 196 nanomoles/ml of lysine. The protein concentration was 2.4 mg/ml (41% recovery). To assay bromoacetylating potential, 1 ml of this solution was incubated at pH 8 with 30 μl of N-acetylcysteamine. After dialysis to remove excess reagent, the solvent was evaporated to yield the title compound. An aliqout of the sample was acid hydrolyzed and assayed by Spinco, revealing 54 nanomoles/ml S-carboxymethyl cysteamine and 140 namomoles/ml lysine (SCMC/lys = 0.38). This indicates that 38% of the lysines present are bromoacetylating moieties.

EXAMPLE 15

Preparation of the cystamine (Bis-2-aminoethyl disulfide) derivative of PRP, (PRP-cys-$NH_2$):

The tetrabutyl ammonium salt of PRP (300 mg) was dissolved in 11 ml of dimethyl formamide (DMF). Subsequently, 30 mg of carbonyldiimidazole was added and the solution stirred at room temperature (r.t.) for 1 hour. The DMF solution was then added with stirring to a chilled (ice) solution of cystamine dihydrochloride (450 mg/10ml $H_2O$; pH adjusted to 10.3 with NaOH) and stirring continued for 15 min. in the ice bath. The solution was then removed from the ice bath and aged at room temperature for 45 min. after transfer to a dialysis bag. Dialysis against (vs.) successive buffer changes was then effected as follows:

(a) vs. 4L pH 7, 0.1M $PO_4$ for 4.2 hours

(b) vs. 4L pH 7, 0.01M $PO_4$ for 8 hours;

(c) vs. 4L pH 7, 0.01M $PO_4$ for 16 hours and

(d) vs. 16L $H_2O$ for 8 hours.

Lyophilization afforded 150 mg of the title compound, cystamine derivative of PRP. The NMR of this material confirmed the cystamine derivatization of PRP. By comparing the two methylene resonances centered at 3 ppm ($CH_2$-S) with the glycosidic proton (5.1 ppm) or the total PRP proton integral, an average value of 48 cystamine moieties per 100 ribosyl-ribitol phosphate moieties (i.e. PRP monomeric unit) was calculated.

EXAMPLE 16

Reduction of PRP-cys-$NH_2$ to Yield PRP-SH:

To 9.0 ml of a pH 8.0 buffer (0.01M in $PO_4$, 0.005M in EDTA, = buffer A) was added 40 mg of PRP-cys-$NH_2$ (see Example 23 for preparation). After complete dissolution (15 min), 42 mg of solid dithiothreitol (DTT) was added. The mixture was degassed, nitrogenated, and aged for 4.5 hours at room temperature. The solution was then transferred to dialysis tubing and dialyzed against the buffer changes as follows:

1) vs. 4L buffer A for 16 hours;
2) vs. 4L buffer A for 7.25 hours;
3) vs. 1L of a pH 8, 0.1M $PO_4$ buffer, 0.005M in EDTA (buffer B) for 17 hours.

At this point an Ellman assay of the solution indicated a thiol titer of 2.04 mmoles SH/ml and therefore a total of 15.3 mmoles of the title PRP-SH for a volume of 7.5 ml.

EXAMPLE 17

Preparation of Maleimidopropionyl-OMPC (MP-OMPC):

10 ml of OMPC(3.2ng/nl) is centrifuged at 43,000RPM, at 4°C for 2 hours and the pellet is resuspended in 8ml of a cooled solution of 1.6mg sodium bicarbonate in 1:1 $H_2O$:MeCN. Maleimidopropionyl N-Hydroxy succinimide (2.6mg) is added and the mixture is agitated for 1 hour on ice. 2ml of 0.1M pH6 phosphate buffer is added and the mixture is centrifuged at 43,000 RPM and 4°C for 2 hours. The maleimidated OMPC is resuspended in 0.01M pH 7 buffer, recentrifuged and resuspended in pH8 0.1M phosphate buffer. The maleimido content is determined by reaction of an aliquot with a known quantity of a N-acetylcysteine followed by Ellman assay.

EXAMPLE 18

Preparation of Thiolated Succinoyl OMPC:

10 ml of OMPC (3.2 mg/ml) is centrifuged at 43,000 RPM and 4°C for 2 hours and the OMPC pellet is resuspended in 6.3 ml of pH 11 borate buffer. Two milliliters of the OMPC suspension are added to each of three tubes which are cooled in ice. An aliquot of succinic anhydride (0.1 M in acetonitrile) is added to each tube with vigorous stirring. Succinic anhydride solution is added to tube #1 (8 µl), tube #2 (16 µl), and tube #3 (32 µl). The reactions are allowed to proceed for 2 hours at 0°C, and one hour at room temperature, followed by an addition to each tube of 2 ml of thiolating solution (prepared by mixing 85 mg EDTA, 17 mg DTT and 46 mg N-acetyl homocysteine thiolactone in 10 ml pH 11 borate buffer). The thiolation reactions are allowed to proceed at room temperature overnight and then each tube is centrifuged at 43,000 RPM, 4°C for 2 hours. The thiolated succinoyl OMPC from each tube is resuspended in 10 ml of pH 8, 0.01 phosphate buffer, recentrifuged and resuspended in 2 ml of pH8 .01 M phosphate buffer. The sulfhydryl titer in each tube is determined by Ellman assay.

EXAMPLE 19 and 20

Synthesis of cyclic gamma aminobutyric acid containing peptides, cPND502 and cPND535 and similar peptides:

The peptides are synthesized by one of two methods: (i) Boc chemistry or (ii) Fmoc chemistry. Both methods are outlined below.

Boc Chemistry: Typically 0.5 mmoles of an amino acid resin is transferred to a reaction vessel and loaded on an automated peptide synthesizer. The Boc chemistry involves removal of the t-butyloxy carbonyl (Boc) group from the α-amino group of the amino acid by trifluoroacetic (TFA) acid. The amino function is then neutralized by diisopropylethylamine (DIEA) after several washes with methylene chloride.

The next amino acid is then coupled to the amino acid on the resin using dicyclohexylcarbodiimide (DCC) and N-hydroxybenzotriazole (HOBt). The cycle is repeated until the peptide of desired length is synthesized. The peptide is cleaved off the resin by using anhydrous hydroflouric acid (HF) in the presence of a scavenger such as anisole. The cleavage is done at approx. 0 degrees centigrade for about 1 hour. The HF is then distilled off and the peptide-resin washed with ether. The peptide is extracted with 10% acetic acid and lyophilized. The dry powder is then purified by reversed-phase HPLC using an appropriate gradient. The peptide is analyzed for purity by analytical HPLC and authenticated by mass spectroscopy.
In all these peptides, the side chain amino function of the lysine residue is blocked by the fluorenylmethyloxycarbonyl (Fmoc) group. This is to prevent the side chain amino function from participating in the cyclization which is the next step.
Cyclization of the peptide is accomplished by dissolving the purified peptide in dimethylformamide (DMF) to a concentration of about 0.001 M. The pH of the solution is adjusted to approximately 8 with DIEA and then 3 equivalents of the BOP reagent is added. The reaction is monitored by HPLC. On completion of the reaction, the DMF is removed on a rotary evaporator and the peptide once again purified by HPLC and then lyophilized. The peptide is then treated with 20% peperidine in DMF to remove the Fmoc group and the peptide is purified by HPLC one more time before being conjugated to a protein and sent for bio-assay.
Fmoc chemistry: The Fmoc chemistry differs from the Boc chemistry only in that the blocking group on the α-amino group is Fmoc and not Boc. The Fmoc group is base labile and therefore is removed by piperidine instead of TFA. The coupling of amino acids is similar to Boc chemistry. The cleavage of the peptide is accomplished by 95% TFA, 2.5% thioanisole & 2.5% ethanedithiol. The cleavage reaction is allowed to go for about 4 to 5 hours at room temperature. The mixture is filtered, the TFA removed on a rotary evaporator and the peptide precipitated in ether. The peptide is purified by HPLC and then cyclized as described above.
According to this description, the following cyclic HIV PND peptides were synthesized:

Ex # : Name : Mass    Sequence

Ex #19 : cPND502 : 1162 : [Seq. Id. : 6 :]

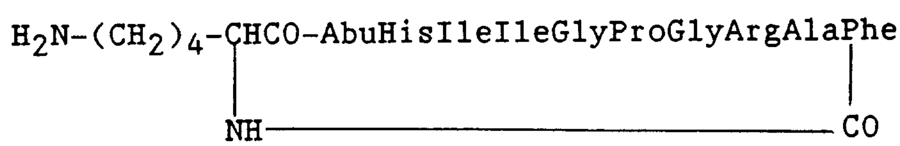

Ex #20 : cPND535 : 1923 : [Seq. Id. : 7 :]

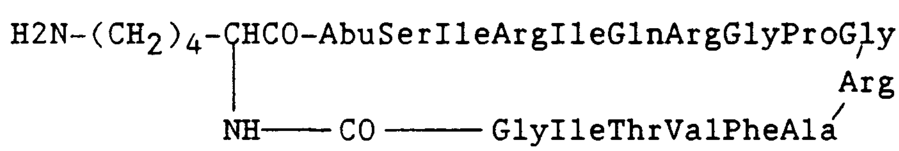

Each of these peptides was maleimidated and conjugated to thiolated OMPC. cPND502-OMPC conjugate provided 13.3% peptide loading, while cPND535 was 12.3% peptide.

While the foregoing specification teaches the principles of the present invention, with examples provided for the purpose of illustration, it will be understood that the practice of the invention encompasses all the usual variations, adaptations, modifications, or deletions as come within the scope of the following claims and its equivalents.

SEQUENCE LISTING

(2) INFORMATION FOR SEQ ID NO:1:

(i) SEQUENCE CHARACTERISTICS:

(A) LENGTH: 11 amino acids

(B) TYPE: amino acid

(D) TOPOLOGY: circular

(ii) MOLECULE TYPE: peptide

(ix) FEATURE:

(A) NAME/KEY: Modified-site

(B) LOCATION: 1

(D) OTHER INFORMATION: /label= Acp and cycle
/note= "6-aminocaproic acid, Acp carboxyl to Lys alpha amino group, and Acp amino group to the free Phe carboxyl"

(ix) FEATURE:

(A) NAME/KEY: Modified-site

(B) LOCATION: 2

(D) OTHER INFORMATION: /label= Nle and cycle

/note= "Lys epsilon amino group to Nle having free amino group for conjugation; Lys alpha amino group to Acp to form cycle"

(ix) FEATURE:

(A) NAME/KEY: Modified-site

(B) LOCATION: 3

(D) OTHER INFORMATION: /label= Acp

/note= "6-aminocaproic acid"

(ix) FEATURE:

(A) NAME/KEY: Modified-site

(B) LOCATION: 11

(D) OTHER INFORMATION: /label= cycle

/note= "Phe carboxyl amide bonded to Acp amino group; Acp carboxyl group amide bonded to Lys alpha amino group to form amide linked cycle"

(xi) SEQUENCE DESCRIPTION: SEQ ID NO:1:

```
Xaa Lys Xaa Gln Arg Gly Pro Gly Arg Ala Phe
1               5                   10
```

(2) INFORMATION FOR SEQ ID NO:2:

(i) SEQUENCE CHARACTERISTICS:

(A) LENGTH: 10 amino acids

(B) TYPE: amino acid

(D) TOPOLOGY: circular

(ii) MOLECULE TYPE: peptide

(ix) FEATURE:

(A) NAME/KEY: Modified-site

(B) LOCATION: 1

(D) OTHER INFORMATION: /label= Acp and cycle

/note= "6-aminocaproic acid; Acp carboxyl group to Lys alpha amino group; Acp amino group to the free Phe carboxyl group"

(ix) FEATURE:

(A) NAME/KEY: Modified-site

(B) LOCATION: 2

(D) OTHER INFORMATION: /label= Nle and cycle

/note= "Lys epsilon amino to Nle having free amino group for conjugation; Lys alpha amino to Acp as the bridge to form cycle"

(ix) FEATURE:

(A) NAME/KEY: Modified-site

(B) LOCATION: 10

(D) OTHER INFORMATION: /label= cycle

/note= "Phe carboxyl group is amide bonded to Acp amino group; Acp carboxyl group is amide bonded to Lys alpha amino group forming amide linked cycle"

(xi) SEQUENCE DESCRIPTION: SEQ ID NO:2:

```
Xaa Lys Asn Arg Gly Pro Gly Arg Ala Phe
1               5                   10
```

(2) INFORMATION FOR SEQ ID NO:3:

(i) SEQUENCE CHARACTERISTICS:
(A) LENGTH: 11 amino acids
(B) TYPE: amino acid
(D) TOPOLOGY: circular

(ii) MOLECULE TYPE: peptide

(ix) FEATURE:
(A) NAME/KEY: Modified-site
(B) LOCATION: 1
(D) OTHER INFORMATION: /label= Acp and cycle
/note= "6-aminocaproic acid, Acp carboxyl group to Lys alpha amino group; Acp amino group to the free Phe carboxyl group"

(ix) FEATURE:
(A) NAME/KEY: Modified-site
(B) LOCATION: 2
(D) OTHER INFORMATION: /label= Nle and cycle
/note= "Lys epsilon amino group to Nle having free amino group available for conjugation; Lys alpha amino group is amide bonded to Acp carboxyl group

(ix) FEATURE:
(A) NAME/KEY: Modified-site
(B) LOCATION: 11
(D) OTHER INFORMATION: /label= cycle
/note= "Phe carboxyl group amide bonded to Acp amino group; Acp carboxyl group amide bonded to Lys alpha amino group to form amide linked cycle"

(ix) FEATURE:
(A) NAME/KEY: Modified-site
(B) LOCATION: 3
(D) OTHER INFORMATION: /label= Acp
/note= "6-aminocaproic acid"

(xi) SEQUENCE DESCRIPTION: SEQ ID NO:3:

```
Xaa Lys Xaa His Ile Gly Pro Gly Arg Ala Phe
1               5                   10
```

(2) INFORMATION FOR SEQ ID NO:4:

(i) SEQUENCE CHARACTERISTICS:
(A) LENGTH: 11 amino acids
(B) TYPE: amino acid
(D) TOPOLOGY: circular

(ii) MOLECULE TYPE: peptide

(ix) FEATURE:
(A) NAME/KEY: Modified-site
(B) LOCATION: 1
(D) OTHER INFORMATION: /label= Aund and cycle
/note= "11-aminoundecanoic acid, Aund carboxyl group to Lys alpha amino group;Aund amino group to the free Phe carboxyl group"

(ix) FEATURE:
(A) NAME/KEY: Modified-site
(B) LOCATION: 2
(D) OTHER INFORMATION: /label= Nle and cycle
/note= "Lys epsilon amino group to a Nle having free amino group available for conjugation; Lys alpha amino amide bonded to Aund carboxyl group to

(ix) FEATURE:
(A) NAME/KEY: Modified-site
(B) LOCATION: 3
(D) OTHER INFORMATION: /label= Aund
/note= "11-aminoundecanoic acid"

(ix) FEATURE:
(A) NAME/KEY: Modified-site
(B) LOCATION: 11
(D) OTHER INFORMATION: /label= cycle
/note= "Phe carboxyl amide bonded to Aund amino group; Aund carboxyl group amide bonded to Lys alpha amino group to form amide linked cycle"

(xi) SEQUENCE DESCRIPTION: SEQ ID NO:4:

```
Xaa Lys Xaa Gln Arg Gly Pro Gly Arg Ala Phe
1               5                   10
```

(2) INFORMATION FOR SEQ ID NO:5:

(i) SEQUENCE CHARACTERISTICS:
(A) LENGTH: 11 amino acids
(B) TYPE: amino acid
(D) TOPOLOGY: circular

(ii) MOLECULE TYPE: peptide

(ix) FEATURE:
(A) NAME/KEY: Modified-site
(B) LOCATION: 1
(D) OTHER INFORMATION: /label= Acp and cycle
/note= "6-aminocaproic acid, Acp carboxyl Lys alpha amino group; Acp amino group is available for amide bond fromation with the free Phe

(ix) FEATURE:
(A) NAME/KEY: Modified-site
(B) LOCATION: 2
(D) OTHER INFORMATION: /label= Nle and cycle
/note= "Lys epsilon amino to Nle having Cbz protected amino group; Lys alpha amino group amide bonded to Acp carboxyl "

(ix) FEATURE:
(A) NAME/KEY: Modified-site
(B) LOCATION: 3
(D) OTHER INFORMATION: /label= Acp
/note= "6-aminocaproic acid"

(ix) FEATURE:
(A) NAME/KEY: Modified-site
(B) LOCATION: 11
(D) OTHER INFORMATION: /label= cycle
/note= "Phe carboxyl is availble for amide bond formation with Acp amino group; Acp carboxyl group is amide bonded to alpha amino of Lys"

(xi) SEQUENCE DESCRIPTION: SEQ ID NO:5:

```
Xaa Lys Xaa Gln Arg Gly Pro Gly Arg Ala Phe
1               5                   10
```

(2) INFORMATION FOR SEQ ID NO:6:

(i) SEQUENCE CHARACTERISTICS:
(A) LENGTH: 10 amino acids
(B) TYPE: amino acid
(D) TOPOLOGY: circular

(ii) MOLECULE TYPE: peptide

(ix) FEATURE:
(A) NAME/KEY: Modified-site
(B) LOCATION: 1
(D) OTHER INFORMATION: /label= cycle
/note= "Lys epsilon amino group available for conjugation; Lys alpha amino is amide bonded to Phe carboxyl group to form cycle"

(ix) FEATURE:
(A) NAME/KEY: Modified-site
(B) LOCATION: 10
(D) OTHER INFORMATION: /label= cycle
/note= "Phe carboxyl group amide bonded to Lys alpha amino group to form amide linked cycle"

(ix) FEATURE:
(A) NAME/KEY: Modified-site
(B) LOCATION: 2
(D) OTHER INFORMATION: /label= Abu
/note= "gamma aminobutyric acid"

(xi) SEQUENCE DESCRIPTION: SEQ ID NO:6:

```
Lys Xaa His Ile Gly Pro Gly Arg Ala Phe
1               5                   10
```

(2) INFORMATION FOR SEQ ID NO:7:

(i) SEQUENCE CHARACTERISTICS:
(A) LENGTH: 18 amino acids
(B) TYPE: amino acid
(D) TOPOLOGY: circular

(ii) MOLECULE TYPE: peptide

(ix) FEATURE:
(A) NAME/KEY: Modified-site
(B) LOCATION: 1
(D) OTHER INFORMATION: /label= cycle
/note= "Lys epsilon amino group available for conjugation; Lys alpha amino group amide bonded to Phe carboxyl group to form a cycle"

(ix) FEATURE:
(A) NAME/KEY: Modified-site
(B) LOCATION: 18
(D) OTHER INFORMATION: /label= cycle
/note= "Gly carboxyl group is amide bonded to Lys alpha amino group to form the amide linked cycle"

(ix) FEATURE:
(A) NAME/KEY: Modified-site
(B) LOCATION: 2
(D) OTHER INFORMATION: /label= Abu
/note= "gamma aminobutyric acid"

(xi) SEQUENCE DESCRIPTION: SEQ ID NO:7:

```
Lys Xaa Ser Ile Arg Ile Gln Arg Gly Pro Gly Arg Ala Phe Val Thr
1               5                   10                  15

Ile Gly
```

## Claims

**1.** A cyclic HIV PND peptide having the structure:

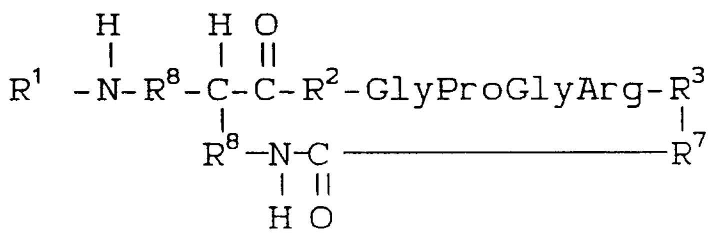

wherein

$R^1$ is:

a) hydrogen, or

b) an amino acid optionally including a marker amino acid selected from norleucine, ornithine, $\beta$-alanine, and gamma amino butyric acid;

$R^2$ is a bond, an amino acid, or a peptide of 2 to 17 amino acids;

$R^3$ is an amino acid or a peptide of 2 to 17 amino acids;

$R^7$ is:

a) a bond, or

b) lower heteroalkylene of formula $-(CH_2)_n-NH-$, wherein n is 1-20;

$R^8$ is a bond or $-(CH_2)_4-$; and

provided that $R^7$ must be $-(CH_2)_n-NH-$ if $R^2$ does not contain the group $-NH-(CH_2)_n-CO-$.

**2.** The cyclic peptide of Claim 1 having the structure:

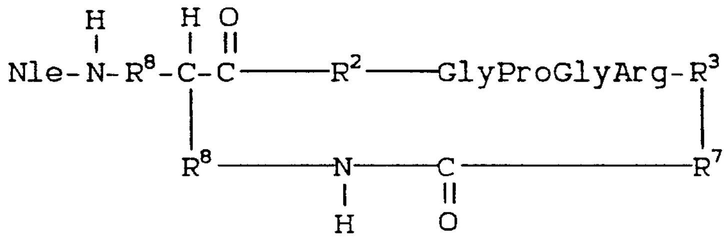

wherein

$R^8$ is a bond or $-(CH_2)_4-$;

$R^7$ is a bond or $-(CH_2)_n$-NH-, wherein n is 1-20;

$R^2$ consists of $X_nX_1X_2$, with $X_2$ closest to the GlyProGlyArg sequence;

$X_1$ is a constituent of $R^2$ selected from:

a) serine,
b) proline,
c) arginine,
d) histidine,
e) glutamine, and
f) threonine;

$X_2$ is a constituent of $R^2$ selected from:

a) isoleucine,
b) arginine,
c) valine,
d) methionine, and
e) -NH-$(CH_2)_n$-CO-, wherein n is 1-20;

$X_n$ is a constituent of $R^2$ and is either a bond, an amino acid, or a peptide of 2 to 15 amino acids, with 0 or one of said amino acids having the structure:

-NH-$(CH_2)_n$-CO-,

wherein n is 1-20;

$R^3$ consists of $X_3X_4X_m$, with $X_3$ closest to the GlyProGlyArg sequence;

$X_3$ is a constituent of $R^3$ selected from:

a) alanine,
b) arginine, and
c) valine;

$X_4$ is a constituent of $R^3$ and is selected from:

a) phenylalanine,
b) isoleucine,
c) valine,
d) leucine;

$X_m$ is a constituent of $R^3$ and is a bond, an amino acid, or a peptide of up to 15 amino acids, with 0 or one of said amino acids having the structure:

-NH-$(CH_2)_n$-CO-,

wherein n is 1-20; and

provided that $R^7$ must be $-(CH_2)_n$-NH- if $R^2$ does not contain the group -NH-$(CH_2)_n$-CO-.

**3.** The cyclic peptide of Claim 2 having the structure:

a) cPND724, [Seq. Id. : 1 :]

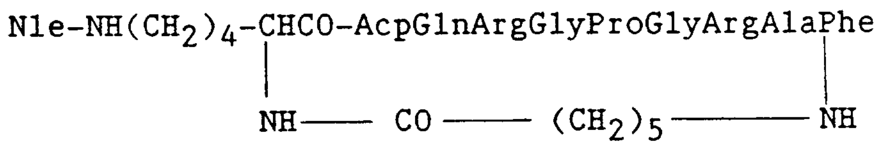

,

b) cPND978, [Seq. Id. : 2 :]

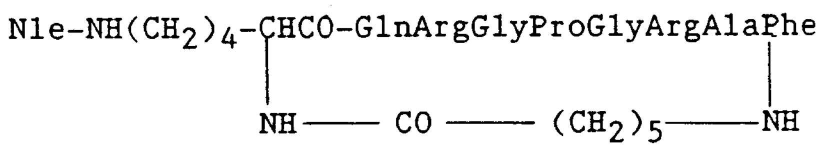

,

c) cPND955 [Seq. Id. : 3 :]

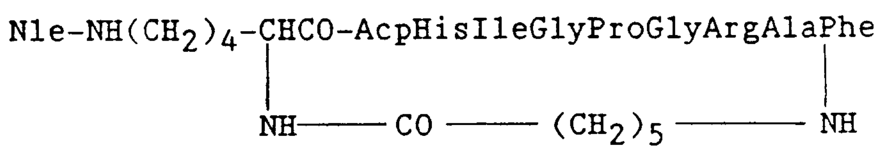

,

d) cPND979, [Seq. Id. : 4 :]

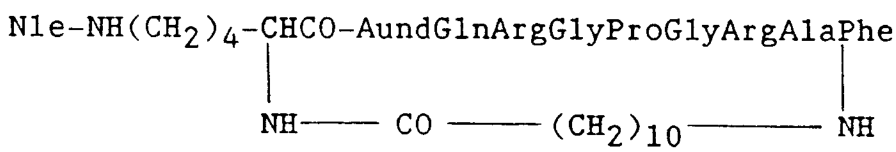

,

e) cPND502, [Seq. Id. : 6 :]

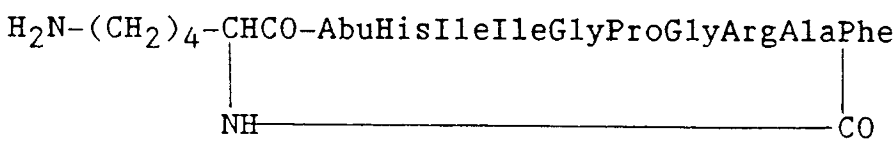

,

or

f) cPND535, [Seq. Id. : 7 :]

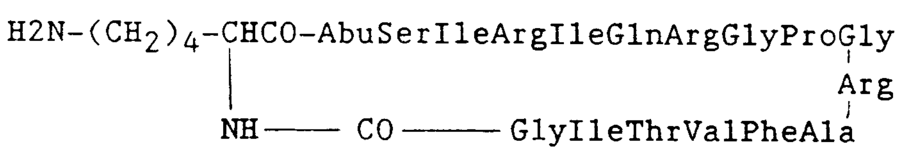

.

**4.** The use of the cyclic HIV PND of Claim 1 for the manufacture of a medicament for inducing mammalian anti-peptide, anti-HIV or HIV-neutralizing immune responses, for preventing HIV-Diseases, and for treating humans afflicted with HIV-Diseases.

5. The use of a conjugate of the cyclic HIV PND of Claim 1 with an immunologic carrier for the manufacture of a medicament for inducing mammalian anti-peptide, anti-HIV or HIV-neutralizing immune responses, for preventing HIV-Diseases, and for treating humans afflicted with HIV-Diseases.

6. A conjugate of the cyclic HIV PND of Claim 1 with an immunologic carrier.